# EUROPEAN PATENT SPECIFICATION

(11) **EP 3 692 964 B1**
(45) Date of publication and mention of the grant of the patent: **03.12.2025**
(21) Application number: 18864109.6
(22) Date of filing: 03.10.2018
(51) Int. Cl.: A61F 13/56, A61F 13/494, A61F 13/62, A61F 13/49, A61F 13/505

(54) **ABSORBENT PAD**
SAUGFÄHIGE UNTERLAGE
GARNITURE ABSORBANTE

(30) Priority: 04.10.2017 JP 2017194463
(43) Date of publication of application: 12.08.2020
(73) Proprietor: Koyo Corporation, Yokohama-shi, Kanagawa 236-0004 (JP)
(72) Inventor: SHIRAI, Atsuko, Yokohama-shi, Kanagawa 236-0004 (JP)
(74) Representative: Samson & Partner Patentanwälte mbB
(86) International application number: PCT/JP2018/037126
(87) International publication number: WO 2019/070009

(56) References cited:
- WO-A1-2009/027872
- WO-A1-2011/014377
- WO-A1-2013/095227
- WO-A1-2013/097878
- WO-A1-2013/097879
- JP-A- 2002 045 396
- JP-A- 2002 500 925
- JP-A- 2006 288 865
- JP-A- 2015 096 112
- JP-B2- 5 833 345

## Description

### TECHNICAL FIELD

The present invention relates to absorbent pads used by being attached to the inner surfaces of a wearing article such as a disposable diaper for adults and an underwear for adults.

### BACKGROUND ART

Absorbent pads such as a urine pad and an incontinence pad are generally used by being attached to the inner surface of a wearing article such as a disposable diaper for adults and an underwear for adults. By exchanging only the absorbent pad that has absorbed urine or the like, the frequency of exchanging diapers and pants is reduced, so the labor burden and the economic cost are reduced.

The absorbent pad generally includes a hook, an adhesive tape, and the like that can be attached to and detached from an inner surface of a wearing article such as a disposable diaper or an underwear. In the following description, the wearer's front-back direction, that is, the direction from the wearer's lower abdomen to buttocks, is the front-back direction and the longitudinal direction of the absorbent pad, and the wearer's left-right direction is the left-right direction and the width direction of the absorbent pad.

Patent Document 1 discloses a conventional absorbent pad that is provided with a large rectangular hooking means in a region having an absorbent core at the center in the front-back direction. However, when the wearing article which attached this absorbent pad is worn, since the both ends of the front-back direction of this absorbent pad are not latched, it is easy to hit a wearer's leg. For this reason, the absorbent pad is likely to be displaced or bent.

Patent Document 1 discloses an improved absorbent pad in which horizontally long belt-like hooking means extending in the left-right direction are provided in a region where there is no absorbent core at both ends in the front-back direction. Further, Patent Document 1 discloses another improved absorption pad, in which one horizontally long belt-like hooking means is provided at both ends in the left-right direction of a region where there is no absorbent core at both ends in the front-back direction. However, since the region provided with the hooking means does not have the absorbent core and has low rigidity, the region is easily bent inside or outside the absorbent pad in the manufacturing process and packaging process of the absorbent pad. In addition, it is not easy to align the entire horizontally long hooking means at both ends in the front-back direction of the absorbent pad, and misalignment tends to occur. Further, when the absorbent pad is repositioned or exchanged while wearing an article of wear, in order to completely peel off the hooking means in the lateral direction, the absorbent pad needs to be separated from the wearing article over substantially the entire lateral direction. Further, since the absorbent pad is twisted when the hooking means is peeled off, the peeled-off hooking means is likely to be mistakenly attached to another part of the wearing article or the absorbent pad itself. When the absorbent pad is peeled in the front-back direction so that the absorbent pad is not twisted, a strong force is required because the horizontally long latching means is peeled off in the vertical direction. It is also difficult to operate with one hand because it is necessary to hold down the wearing article. These tasks are not easy for the elderly and people with disabilities.

Patent Document 2 discloses a disposable auxiliary pad that is used by being attached to a disposable diaper body or a sanitary napkin. The pad is provided with fastening members having a vertically long rectangular shape on both side edges in the width direction of the front portion and the back portion of the pad. The four fastening members are located at least 10 mm inward from the side edge in the width direction of the pad and at least 10 mm inward from the side edge in the width direction of the absorbent body. This is to make it easier to pinch. Further, the interval between the fastening members in the width direction is 50 mm to 90 mm. This is because an operator can insert his / her hand between the fastening members so that the center part in the width direction of the pad can be easily pinched. However, the two fastening members at both side edges in the width direction of the pad are separated from each other so that a hand can be inserted between them. For this reason, both the fastening members cannot be attached to the inner surface of the wearing article with only one hand. When the pad is attached to the inner surface of the wearing article, it is not easy to align the fastening member so that the pad is not bent or wrinkled. If the pad is bent and wrinkled, the excrement may leak. Moreover, in the manufacturing process of a wide pad such as the pad, the pad is generally folded inward at both ends in the width direction, then folded in the length direction, and then packaged. Since the pad is provided with the fastening members at both ends in the width direction, the fastening members are folded when the both ends of the pad are folded inward. For this reason, the pad cannot be attached unless the pad is completely spread out and the fastening members at both ends are pressed, and the attaching operation is complicated.
WO 2013/097878 A1 relates to an absorbent article comprising a body facing surface and a garment facing surface. A mechanical fastening material covering the whole garment facing surface is shown. Also, expandable ink has been applied around the whole circumference of the mechanical fastening material. WO 2009/027872 A1 relates to an undergarment attached absorbent article that comprises a liquid permeable topsheet, a generally liquid impermeable backsheet, and an absorbent core positioned between the backsheet and the topsheet.

### RELATED ART DOCUMENTS

### PATENT DOCUMENTS

Patent document 1: Japanese Patent Application Publication No. 2000-139982
Patent document 2: Japanese Patent No. 5833345

### SUMMARY OF THE INVENTION

### PROBLEMS TO BE SOLVED BY THE INVENTION

In view of the above circumstances, the present invention has an object to provide an absorbent pad that can be easily attached and positioned on a wearing article.

### MEANS OF SOLVING THE PROBLEMS

According to the present invention, an absorbent pad with a longitudinal direction as a front-back direction of a wearer includes a liquid-retaining absorber with a longitudinal direction as the front-back direction; a liquid-permeable top sheet covering an upper surface of the absorber; a liquid-impermeable bottom sheet covering a lower surface of the absorber; a wearing-article-facing surface on a side opposite to the absorber-facing surface of the bottom sheet; left and right rising sheets provided with rising portions that are provided with an elastic body to expand and contract in the front-back direction and rise along left and right ends of the top sheet, the left and right rising sheets being fixed with an adhesive near both ends of the left-right direction and near both ends of the front-back direction of the top sheet except for the rising portions; a hook member having a length in the front-back direction of 20 mm or more and 50 mm or less and a width in the left-right direction of 10 mm or more and 20 mm or less, the hook member including a collection of hook pieces that are detachably hooked on a wearing article; and a hook member fixing region having a length in the front-back direction of 20 mm or more and 50 mm or less and a width in the left-right direction of more than 30 mm and less than 70 mm, and in which a plurality of the hook members are fixed parallel at intervals of less than 50 mm in the left-right direction of the absorbent pad, wherein the hook member fixing region is a region obtained by projecting each center region in the left-right direction of both a front end portion and a back end portion of the absorber onto the wearing-article-facing surface of the bottom sheet, and wherein the absorbent pad is folded in half at a front-back center line of the absorbent pad as a fold so that the hook member is arranged outside before use.

According to another aspect of the present invention, a sheet having the hook member fixed to a surface is fixed to the hook member fixing region. According to still another aspect of the present invention, left and right side portions of the absorbent pad are folded so as to overlap the top sheet at crease lines along outer edge of each tip of the left and right rising sheets that overlap the top sheet.

According to still another aspect of the present invention, the hook member is fixed to the hook member fixing region with an adhesive, and the adhesive is applied to an entire region along a side edge farther from a left-right center line of the absorbent pad, and the adhesive is not applied to a region along a side edge nearer the left-right center line of the absorbent pad.

The present invention is not limited to the above aspect. Further details, features, functions and effects of the present invention will be more apparent from the following description of preferred exemplary embodiments of the present invention.

### BRIEF DESCRIPTION OF THE DRAWINGS

FIG. 1 is a bottom view of an absorbent pad according to an embodiment of the present invention in a state where an elastic body does not exert contraction force;
FIG. 2 is a schematic cross-sectional view of the absorbent pad with the top sheet disposed above, taken along line II-II in FIG. 1;
FIG. 3 is a schematic cross-sectional view of the absorbent pad with the top sheet disposed above, taken along line III-III in FIG. 1;
FIG. 4 is a bottom view of an absorbent pad according to another embodiment of the present invention in a state where an elastic body does not exert contraction force;
FIG. 5 is a schematic cross-sectional view of the absorbent pad with the top sheet disposed above, taken along line V-V in FIG. 4;
FIG. 6 is a bottom view of an absorbent pad according to still another embodiment of the present invention in a state where an elastic body does not exert contraction force; and
FIG. 7 is a schematic cross-sectional view of the absorbent pad with the top sheet disposed above, taken along line VII-VII in FIG. 6.

### MODE FOR CARRYING OUT THE INVENTION

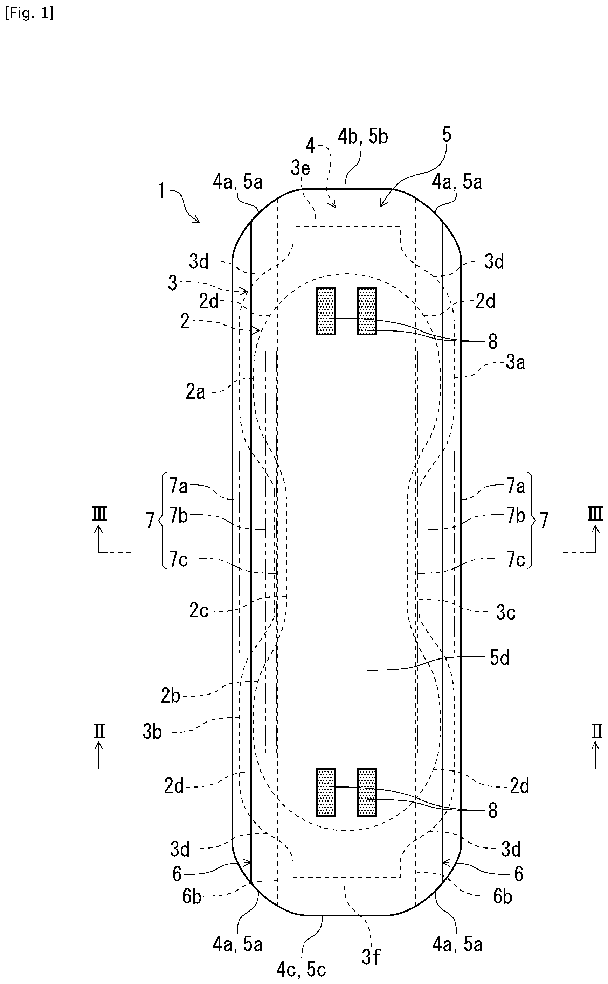

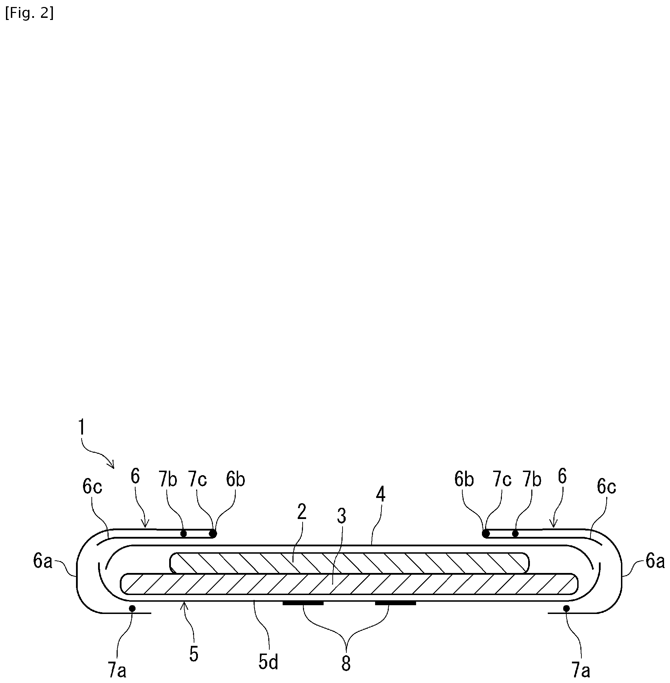

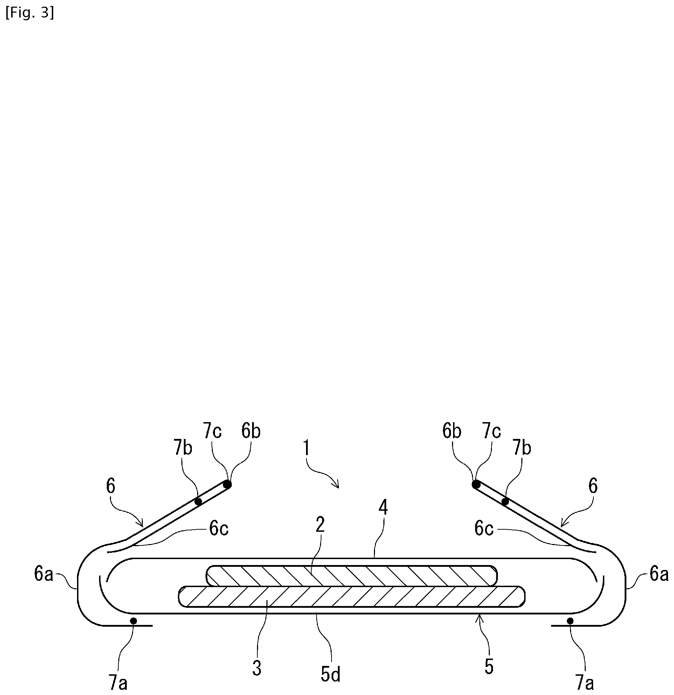

With reference to FIGS. 1 to 3, an absorbent pad 1 according to an embodiment of the present invention will be described. The absorbent pad 1 is attached to the inner surface of a wearing article (not shown) such as a disposable diaper or underwear for adults and is used as an incontinence pad and a urine pad. The front-back direction of the wearer, that is, the direction from the lower abdomen to the buttocks of the wearer is defined as the front-back direction and longitudinal direction of the absorbent pad 1. The absorbent pad 1 includes an upper absorber 2, a lower absorber 3, a top sheet 4, a bottom sheet 5, a wearing-article-facing surface 5d, a rising sheet 6, an elastic body 7, and a hook member 8.

The absorbers 2 and 3 are liquid-retaining properties that quickly absorb body fluids such as urine and hold them inside. The front-back direction of the wearer is the front-back direction and longitudinal direction of the absorbers 2 and 3. The absorbers 2 and 3 respectively have front portions 2a and 3a corresponding to the lower abdomen of the wearer, back portions 2b and 3b corresponding to the buttocks of the wearer, and intermediate portions 2c and 3c corresponding to the crotch of the wearer. The absorbers 2 and 3 respectively have rounded corners 2d, 2d, 2d, 2d and 3d, 3d, 3d, 3d. The width of the intermediate portion 2c in the front-back direction of the upper absorber 2 is substantially constant and is narrower than the maximum widths of the front portion 2a and the back portion 2b. The width of the intermediate portion 3c in the front-back direction of the lower absorber 3 is substantially constant and is narrower than the maximum widths of the front portion 3a and the back portion 3b.

The front portion 2a and the back portion 2b of the upper absorber 2 have substantially the same shape and size. The front portion 3a and the back portion 3b of the lower absorber 3 have substantially the same shape and size. Therefore, the absorbent pad 1 can be used without distinguishing the front and back. The shapes and sizes of the front portions 2a and 3a and the back portions 2b and 3b of the absorbers 2 and 3 are not limited to the above and may vary depending on the type and size of the absorbent pad.

The maximum length in the front-back direction of the lower absorber 3 is the length between the front end 3e and the back end 3f of the middle portion in the left-right direction. The maximum width in the left-right direction of the lower absorber 3 is the width of the intermediate region in the front-back direction of the front portion 3a corresponding to the lower abdomen of the wearer and the width of the intermediate region in the front-back direction of the back portion 3b corresponding to buttocks of the wearer. However, the width is not limited to the above, and the width of the front end 3e and the back end 3f may have the same width as the intermediate portion in the front-back direction.

The entire upper absorber 2 is within the contour of the lower absorber 3 and overlaps the lower absorber 3. The absorber of the present invention is not limited to the two-layer structure as described above, but may be a single-layer structure composed of only one absorber, a structure in which three or more absorbers are overlapped, or a structure in which a plurality of absorbers are partially overlapped.

The absorbers 2 and 3 are manufactured, for example, by a method in which water absorbent fibers obtained by defusing a roll-sheet pulp and powdery or granular super absorbent polymers are pneumatically conveyed to a mold provided on the peripheral surface of the suction drum and are sucked and deposited into the mold and are molded, and then the molded is wrapped with water absorbent paper or non-woven fabric (not shown). The materials of absorber 2 and 3 are the same, but not limited to this. Different types and blending ratios of the materials of the upper absorber 2 and the lower absorber 3 may be used so that each has more appropriate functions and effects.

The top sheet 4 covers the upper surface of the entire absorbers 2 and 3. The front-back direction and longitudinal direction of the top sheet 4 are the front-back direction of the wearer. The top sheet 4 has rounded corners 4a, 4a, 4a, 4a. The maximum length in the front-back direction of the top sheet 4 is the length between the front end 4b and the back end 4c at the center of the left-right direction. The top sheet 4 is placed facing the skin of the wearer. The top sheet 4 has liquid permeability. The discharged urine or other liquid material passes through the top sheet 4 and moves to the absorbers 2 and 3. The top sheet 4 is made of a liquid permeable non-woven fabric, for example, a non-woven fabric made of chemical fibers such as polyethylene, polypropylene, polyester, nylon and rayon, natural fibers such as cotton and cellulose, or a combination thereof. The top sheet 4 is not limited to the non-woven fabric but may be made of a liquid-permeable material such as a perforated film, tissue, or a laminate thereof, or a sheet obtained by appropriately connecting them.

The bottom sheet 5 covers the lower surface of the entire absorbers 2 and 3. The front-back direction and longitudinal direction of the bottom sheet 5 are the front-back direction of the wearer. The bottom sheet 5 has rounded corners 5a, 5a, 5a, 5a. The maximum length in the front-back direction of the bottom sheet 5 is the length between the front end 5b and the rear end 5c at the center of the left-right direction. The bottom sheet 5 has liquid impermeability and is made of a liquid-impermeable material such as a polyethylene film, for example. The bottom sheet 5 prevents the liquid that has passed through the top sheet 4 or the liquid that the absorbers 2 and 3 have not absorbed or retained from leaking to the outside.

The surface of the bottom sheet 5 opposite to the surface facing the absorbers 2 and 3 is a wearing-article-facing surface 5d that faces a wearing article such as a disposable diaper or underwear.

The front-back direction of rising sheet 6 is the longitudinal direction. The left and right rising sheets 6 and 6 have portions that are provided with the elastic body 7 (described later) to expand and contract in the front-back direction and that rise along both left and right end rigions of the top sheet 4. The left and right rising sheets 6 and 6 are fixed to the region along the left-right direction both ends of the wearing-article-facing surface 5d of the bottom sheet 5 with an adhesive or the like. The left and right rising sheets 6 and 6 are folded up above the top sheet 4 at fold lines 6a and 6a along both ends of the left-right direction of the top sheet 4 and bottom sheet 5. The rising sheets 6 and 6 have folded portions 6c and 6c that are folded toward the top sheet 4 at the folds 6b and 6b at the tip. The left and right rising sheets 6 and 6 are fixed with an adhesive or the like near both ends of the left-right direction and near both ends of the front-back direction of the top sheet 4 except for the rising portions.

The rising sheet 6 has liquid impermeability and is made of a nonwoven fabric, for example, a nonwoven fabric made of chemical fibers such as polyethylene, polypropylene, polyester, nylon, rayon, natural fibers such as cotton and cellulose, or a combination thereof. The rising sheet 6 is not limited to the non-woven fabric, and may be made of, for example, a resin film, a tissue, an elastic material, a laminate thereof, or a sheet obtained by appropriately connecting them.

The left and right rising sheets 6 and 6 are provided with a plurality of elastic bodies 7 that expand and contract in the front-back direction. The elastic bodies 7a and 7a are disposed between the front portion 3a and the back portion 3b of the lower absorber 3 along the vicinity of the folds 6a and 6a at the root of the left and right rising sheets 6 and 6. The elastic bodies 7c and 7c are disposed between the center of the front-back direction of the front portion 3a to the center of the front-back direction of the back portion 3b of the lower absorber 3 along the inner edge of the folds 6b and 6b at the tip of the left and right rising sheets 6 and 6. The elastic bodies 7b and 7b have substantially the same length in the front-back direction as the elastic bodies 7c and 7c, and are disposed in parallel with an interval on the outer side in the left-right direction of the elastic bodies 7c and 7c. The number and position of the elastic body 7 are not limited to the above. The left and right rising sheets 6 and 6 are fixed near both ends of the left-right direction and near both ends of the front-back direction of the top sheet 4. When the rising sheets 6 and 6 provided with the elastic body 7 are contracted by the contraction force of the elastic body 7, the leading portions with the folds 6b and 6b of the rising sheets 6 and 6 stand up to form standing gather units. The standing gather units are close to the crotch of the wearer and prevent excrement etc. from leaking sideways.

The elastic body 7 is made of a thread-like stretchable material such as natural rubber, polyurethane resin, stretchable hot melt, and the like. The elastic body 7 is not limited to the thread-like elastic body and may be a band-like or sheet-like elastic body that expands and contracts in the front-back direction at an appropriate position and range of the rising sheets 6 and 6.

The hook member 8 is provided with a collection of hook pieces that are detachably hooked on the surface of a wearing article such as a disposable diaper or underwear. A suitable hook member known as a hook-and-loop fastener may be used. The two hook members 8 and 8 that are long in the front-back direction are arranged in parallel with an interval in the left-right direction of the absorbent pad 1. The number of the hook members 8 arranged in parallel is not limited to two and may be three or more. When the two hook members 8 and 8 are arranged in parallel in the left-right direction with an interval, the length in the front-back direction of each hook member 8 is 20 mm or more and 50 mm or less, preferably 25 mm or more and 40 mm or less. The width in the left-right direction of each hook member 8 is shorter than the length in the front-back direction and is 10 mm or more and 30 mm or less, preferably 10 mm or more and 20 mm or less. Since the longitudinal direction of the hook member 8 matches the front-back direction of the absorbent pad 1, the hook member 8 can be easily aligned and attached, and the hook member 8 can be removed with a small force. Moreover, since the relatively short hook members 8 are arranged in parallel with an interval, they can be stably attached to the wearing article and can be easily removed.

The hook member 8 is fixed to the hook member fixing region in the wearing-article-facing surface 5d of the absorbent pad 1 by an adhesive or heat welding. The hook member fixing region of the absorbent pad 1 is a region obtained by projecting each center region in the left-right direction of both a region near the front end 3e of the front portion 3a and a region near the back end 3f of the back portion 3b onto the wearing-article-facing surface 5d of the bottom sheet 5. The hook member fixing region overlaps with a region obtained by projecting each center region in the left-right direction of both a front end region of the front portion 2a and a back end region of the back portion 2b onto the wearing-article-facing surface 5d of the bottom sheet 5. When a plurality of absorbers are partially or wholly overlapped, the hook member fixing region is preferably a region obtained by projecting each center region in the left-right direction of both a front end region of the front portion and a back end region of the back portion of the overlapped absorbers onto the wearing-article-facing surface 5d of the bottom sheet 5.

The hook member fixing region to which the hook member 8 is fixed is preferably smaller than the size of a general adult palm. The length in the front-back direction of the hook member fixing region is 20 mm or more and 50 mm or less, preferably 25 mm or more and 40 mm or less. The width in the left-right direction of the hook member fixing region is more than 30 mm and less than 70 mm, preferably more than 50 mm and less than 65 mm. The absorbent pad 1 has the hook member fixing region which is arranged in each center region in the left-right direction of both the front end region of the front portion and the rear end region of the back portion of the absorber. The absorbent pad 1 has the length in the front-back direction and the width in the left-right direction of the hook member fixing region are within the above range. When the absorbent pad 1 is folded in two along the front-back center line, the hook members 8 fixed to the front end and back end can be covered with one hand at the same time. When the absorbent pad 1 is inserted inside the pulled down underwear, the hook member 8 is not caught. When the hook member 8 is aligned with the wearing article and pressed with the palm of one hand from the top sheet 4, the hook member 8 is easily hooked to the wearing article, and the absorbent pad 1 is attached to the wearing article. Furthermore, the absorbent pad 1 can be peeled off the wearing article with a light force by holding one end of the front-back direction with one hand. At this time, the absorbent pad 1 is peeled upward in the direction opposite to the wearing article, so that the hook member 8 is not accidentally hooked to another part of the wearing article. Therefore, the absorbent pad 1 can be easily replaced even by elderly people or persons with disabilities.

The hook member of the present invention is not limited to a shape that is long in the front-back direction of the absorbent pad 1. For example, a unit in which a plurality of hook members having a substantially square shape are arranged in a line with an interval in the front-back direction of the absorbent pad 1 may be arranged in parallel with an interval in the left-right direction of the absorbent pad 1. The hook member fixing region in which the hook members are fixed as described above is also preferably smaller than the size of a general adult palm.

When each elastic body 7 contracts by exerting a contraction force, the portions where the elastic bodies 7a and 7a of the left and right rising sheets 6 and 6 are arranged become the roots, and the tip portions where the elastic bodies 7b and 7b and the elastic bodies 7c and 7c are arranged in the left and right rising sheets 6 and 6 rise to form the standing gather units. Further, the tip portions of the rising sheets 6 and 6 are contracted in the front-back direction, and the four corners 4a and 5a of the absorbent pad 1 are pulled. At this time, since the upper absorber 2 and the lower absorber 3 of the absorbent pad 1 have rounded corners 2d and 3d, the front end region, the rear end region, the left end region and the right end region of the absorbent pad 1 become a gently curved shape with the bottom sheet 5 convex. As a result, hook members 8 and 8 fixed to the hook member fixing region in the wearing-article-facing surface 5d of the bottom sheet 5 protrude toward the wearing article. In particular, the hook member fixing region overlaps the region where the entire upper absorber 2 and the lower absorber 3 are overlapped, and the thickness and rigidity are increased. Since the corners of the upper absorber 2 are rounded, the entire absorbent pad 1 is curved more greatly. Therefore, hook members 8 and 8 fixed to the hook member fixing region of the wearing-article-facing surface 5d protrude toward the wearing article, and the absorbent pad 1 of the present invention is easy to align and attach to the wearing article. Further, not only the corners 2d and 3d of the absorbers 2 and 3, but also the corners at both ends of the front-back direction of the absorbent pad 1 are cut into rounded shapes. In other words, the corners of the top sheet 4, the bottom sheet 5, and the rising sheets 6 and 6 are rounded in the same shape. Therefore, for example, when the absorbent pad 1 is mounted between the right and left raised gathers of the disposable diaper, the corners do not bend or kink, so that reworking is unnecessary and mounting is easier.

The absorbent pad of the present invention can be produced by combining various known methods for continuously manufacturing absorbent pads and a method for producing hook members described below. According to one method of manufacturing a hook member of the present invention, in a continuous manufacturing process of the absorbent pad 1, a tape in which the hook member with an adhesive is continuous is conveyed, and the tape is divided into two at the center line in the width direction. The two tapes with the adhesive are conveyed and cut to a desired length to obtain the two hook members 8 and 8. The two hook members 8 and 8 with the adhesive are fixed to the hook member fixing region in the wearing-article-facing surface 5d of the bottom sheet 5. Thus, the absorbent pad 1 provided with the hook member 8 of the present invention can be easily manufactured. According to this manufacturing method, the number and length of the hook members to be fixed to the absorbent pad can be easily and appropriately changed so that the hook member can obtain an appropriate engagement strength according to the type and size of the absorbent pad. Even when the type of the hook member is changed and the engagement strength per area is changed, the number and length of the hook members are appropriately changed, so that the engagement force for pad attachment can be adjusted and the cost can be further reduced.

When manufacturing an absorbent pad with the three hook members 8, 8 and 8 fixed in the hook member fixing region, in the above manufacturing process, the tape with a continuous hook member with an adhesive is divided into three equal parts in the width direction. Then, the three tapes with the adhesive can be cut to a desired length to obtain the three hook members.

According to another one of the manufacturing methods of the absorbent pad of the present invention, in the process of continuously manufacturing the absorbent pad 1, the tape in which the hook members are continuous is conveyed, and an adhesive is applied to an area approximately 1 to 3 mm inside from both end portions in the width direction on the back surface of the tape. The tape to which the adhesive is applied except for both end areas in the width direction is conveyed and is divided into two at the center line in the width direction. The two tapes are crossed on the transport line and the left and right sides are switched so that the areas where the adhesive of the two tapes is applied are arranged at both ends in the width direction of the transport line. The two tapes are conveyed and are cut to a desired length to obtain the two hook members 8 and 8. The two hook members 8 and 8 are fixed to the hook member fixing region in the wearing-article-facing surface 5d of the bottom sheet 5, while the adhesive application area is placed on both ends in the width direction of the hook member fixing region.

According to this method of manufacturing the hook member, since an expensive hook tape with an adhesive is not used, the manufacturing cost can be reduced. The adhesive is applied to the area approximately 1 to 3 mm inside from both end portions in the width direction on the back surface of the tape and can be prevented from protruding from the tape. Further, according to the manufacturing method, the hook member is fixed to the hook member fixing region with the adhesive, and the adhesive is applied to the entire region along the side edge far from the left-right center line of the absorbent pad, and the adhesive is not applied to the region along the side edge near the left-right center line of the absorbent pad. Each hook member has the area where the adhesive is entirely applied on the outside and the area where the adhesive is not applied on the inside. Therefore, it is possible to reduce the risk that the area where the adhesive is not applied is lifted or peeled off and touch and damage the skin of the wearer.

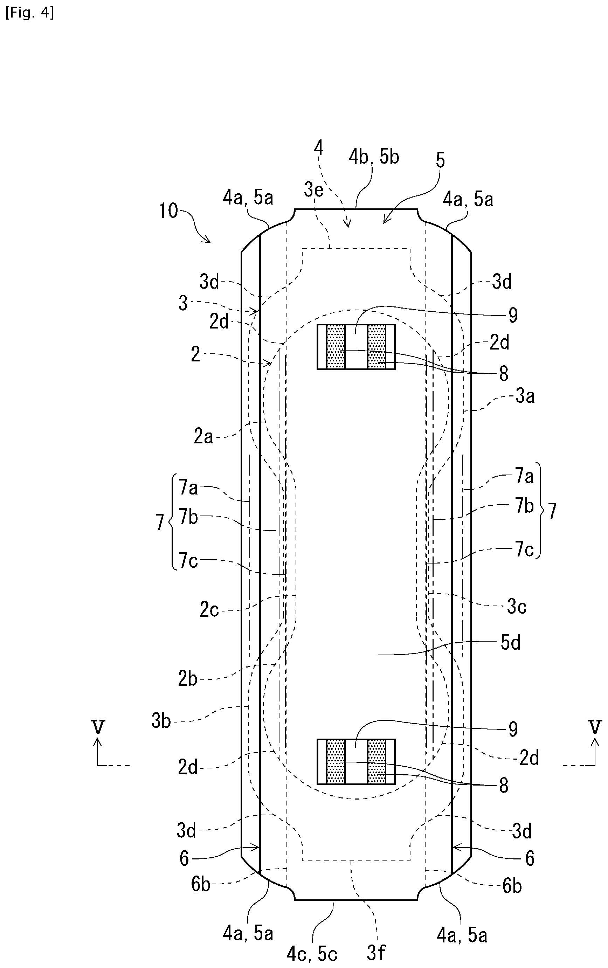

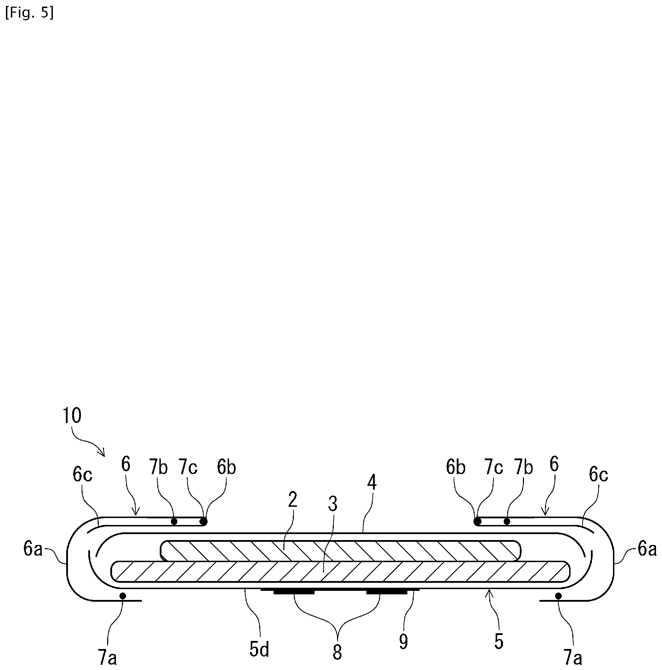

Next, with reference to FIGS. 4 and 5, an absorbent pad 10 according to another embodiment of the present invention will be described. Like the absorbent pad 1, the absorbent pad 10 is attached to the inner surface of a wearing article such as an disposable diaper or underwear for adults (not shown) and used as an incontinence pad and a urine pad. The absorbent pad 10 includes the upper absorber 2, the lower absorber 3, the top sheet 4, the bottom sheet 5, the wearing-article-facing surface 5d, the rising sheets 6 and 6, the elastic body 7, and the hook member 8. Since the structure and function of these elements are the same as those of the absorber 1, description thereof is omitted. Unlike the absorbent pad 1, the absorbent pad 10 includes a sheet 9 for hook members.

As shown in FIGS. 4 and 5, the two hook members 8 and 8 that are long in the front-back direction are fixed in parallel at an interval on the surface of the sheet 9 for hook members of the absorbent pad 10. The sheet 9 for hook members to which the hook members 8 and 8 are fixed is fixed to the hook member fixing region of the wearing-article-facing surface 5d of the absorbent pad 10 with an adhesive or the like. The hook member sheet 9 is fixed to the wearing-article-facing surface 5d so that the hook member 8 is disposed in the hook member fixing region described above. Since the shape, number, structure, arrangement, and function of the hook member 8 and the hook member fixing region can be the same as those of the absorbent pad 1, description thereof is omitted.

The sheet 9 for hook members is made of, for example, a resin film such as a polyethylene film or a non-woven fabric. The sheet 9 for hook members is not limited to the one described above, and may be bonded to both the hook member 8 and the bottom sheet 5 with an adhesive or the like, and may be made of a material having such a strength that it is not broken or peeled off when the hook member is removed from the wearing article.

As a method for manufacturing the absorbent pad 10, for example, two long hook tapes with an adhesive, which is the material of the hook member 8, are fixed in parallel at the desired interval on the surface of the long sheet, which is the material of the sheet 9 for hook members. Then, the sheet with hook members is prepared in advance. The sheet with hook members is cut to a desired length according to the type and size of the absorbent pad, and the sheet 9 for hook members to which the two hook members 8, 8 are fixed are obtained. The sheet 9 is fixed to the hook member fixing region of the bottom sheet 5 with an adhesive or the like, and the absorbent pad 10 is manufactured. According to this manufacturing method, the absorbent pad 10 can be easily manufactured by preparing in advance the sheet 9 for hook members in which a plurality of the hook members are fixed in parallel and bonding them to the bottom sheet 5.

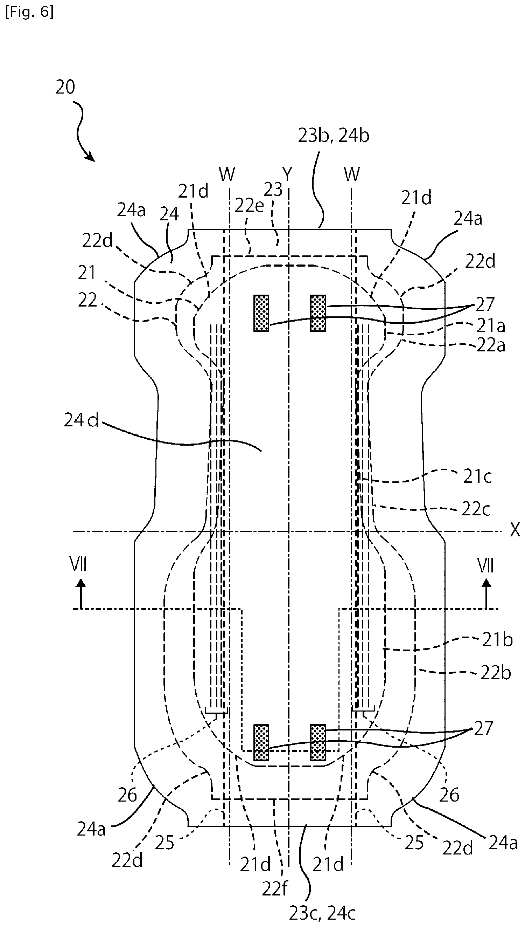

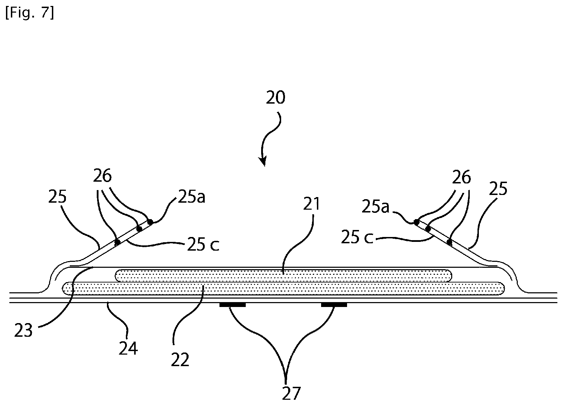

Next, with reference to FIGS. 6 and 7, an absorbent pad 20 according to still another embodiment of the present invention will be described. Like the absorbent pad 1, the absorbent pad 20 is attached to the inner surface of a wearing article such as a disposable diaper or underwear for adults (not shown) and used as an incontinence pad and a urine pad. The front-back direction and longitudinal direction of the absorbent pad 20 are the front-back direction of the wearer, that is, the direction from the lower abdomen to the buttocks of the wearer. In FIG. 6, X is a front-back center line of the absorbent pad 20, Y is a left-right center line of the absorbent pad 20, and W and W are left and right folding lines of the absorbent pad 20. The absorbent pad 20 includes an upper absorber 21, a lower absorber 22, a top sheet 23, a bottom sheet 24, a wearing-article-facing surface 24d, a rising sheet 25, an elastic body 26, and a hook member 27. Regarding the description of each element of the absorbent pad 20, the difference from the element of the same name of the absorbent pad 1 will be mainly described, and the description of the common points will be omitted.

The entire upper absorber 21 is within the contour of the lower absorber 22 and overlaps the lower absorber 22. The absorbers 21 and 22 have front portions 21a and 22a corresponding to the lower abdomen of the wearer, back portions 21b and 22b corresponding to the buttocks of the wearer, and intermediate portions 21c and 22c corresponding to the crotch of the wearer. The absorbers 21 and 22 have rounded corners 21d, 21d, 21d, 21d and 22d, 22d, 22d, 22d. The front end 22e and the rear end 22f of the lower absorber 22 have a narrow width in the left-right direction. The length in the front-back direction of the front portions 21a and 22a of the absorbers 21 and 22 is less than half the length in the front-back direction of the back portions 21b and 22b. The maximum width in the left-right direction of the front portion 21a of the upper absorber 21 is substantially the same as the maximum width in the left-right direction of the back portion 21b. The width in the left-right direction of the intermediate portion in the front-back direction is substantially constant and narrower than any of the maximum widths in the left-right direction of the front portion 21a and the back portion 21b. The maximum width in the left-right direction of the front portion 22a of the lower absorber 22 is narrower than the maximum width in the left-right direction of the back portion 22b. The width in the left-right direction of the intermediate portion in the front-back direction is substantially constant and narrower than any of the maximum widths in the left-right direction of the front portion 22a and the back portion 22b. The absorbers 21 and 22 can be manufactured by the same material and manufacturing method as the absorbers 2 and 3.

The top sheet 23 has liquid permeability like the top sheet 4 and can be manufactured using the same material. The top sheet 23 has a substantially rectangular shape that is long in the front-back direction, covers the upper surfaces of the absorbers 21 and 22, and is arranged toward the skin of the wearer. The bottom sheet 24 has a substantially gourd shape in which the intermediate portion in the front-back direction is narrowed, covers the entire lower surface of the absorbers 21 and 22, and is arranged toward the wearing article. The bottom sheet 24 has rounded corners 24a, 24a, 24a, 24a. The bottom sheet 24 has liquid impermeability like the bottom sheet 5 and can be made of the same material. The surface of the bottom sheet 24 opposite to the surface that faces the absorbers 21 and 22 is a wearing-article-facing surface 24d that faces a wearing article such as a disposable diaper or underwear.

The left and right rising sheets 25 and 25 have portions that are provided with the elastic body 26 (described later) to expand and contract in the front-back direction and that rise along both left and right end rigions of the top sheet 23. The left and right rising sheets 25 and 25 are fixed to the region along the left-right direction both ends of the wearing-article-facing surface 24d of the bottom sheet 24 with an adhesive or the like. The rising sheets 25 and 25 have folded portions 25c and 25c that are folded toward the top sheet 23 at the folds 25b and 25b at the tip. The left and right rising sheets 25 and 25 are fixed with an adhesive or the like near both ends of the left-right direction and near both ends of the front-back direction of the top sheet 23 and the bottom sheet 24 except for the rising portions. The rising sheet 25 has liquid impermeability like the rising sheet 6 and can be made of the same material as the rising sheet 6.

A plurality of elastic bodies 26 extending and contracting in the front-back direction are fixed with an adhesive or the like between the vicinity of the front ends of the left and right rising sheets 25 and 25 and the folded portions 25c and 25c. When the rising sheets 25 and 25 are contracted by the contraction force of the elastic body 26, the leading portions of the rising sheets 25 and 25 stand up to form standing gather units. The standing gather units are close to the crotch of the wearer and prevent excrement etc. from leaking sideways. The elastic body 26 has the same elasticity as the elastic body 7 and can be made of the same material as the elastic body 7.

The hook member 8 and hook member fixing region of the absorbent pad 20 have the same shape, structure and arrangement as those of the absorbent pad 1 and can be made of the same material. When the absorbent pad 20 is attached to the inner surface of the wearing article, the absorbent pad 20 is folded at the left and right folding lines W and W along the outer edges of the leading portions of the right and left rising sheets 25 and 25 overlapped on the top sheet 23 as folds so that the left and right sides of the absorbent pad 20 overlap the top sheet 24. Further, the absorbent pad 20 is folded in half at the front-back center line X or its vicinity as a fold so that the hook member 8 is arranged outside. The absorbent pad 20 is preferably sold in such a folded state.

When the absorbent pad 20 is attached to the inner surface of a wearing article worn by a seated wearer, the wearing article is lowered to the wearer's knee, and then the folded absorbent pad 20 has its front and rear end portions hold with one hand and is inserted into the wearing article. At that time, the hook members 8 and 8 at the ends can be covered with one hand, so that the hook member 8 can be arranged to face the inner surface of the wearing article without hooking the hook member 8 on the wearing article. Then, the absorbent pad 20 is spread on the inner surface of the wearing article, and the hook member 8 is aligned with the wearing article and pressed with the palm of one hand from the top sheet 4 side. Thus, it is possible to easily hook the hook member 8 to the wearing article and attach the absorbent pad 20 to the wearing article.

The present invention is not limited to the embodiments described above. The scope of the present invention includes at least modifications that are obvious from the above-described embodiments and modifications that do not change the essence of the present invention. For example, the absorbent pad 20 may not be folded at the left and right folding lines W and W so that the left and right portions are overlapped over the top sheet 24. Instead, the absorbent pad may be folded in half at the front-back center line X or the vicinity thereof as a fold so that the hook member is arranged outside.

### INDUSTRIAL APPLICABILITY

The absorbent pad of the present invention can be attached to the inner surface of a wearing article such as a disposable diaper or underwear for adults and used as an incontinence pad or urine pad.

### DESCRIPTION OF THE REFERENCE NUMERAL

1,10,20: absorbent pad,
2,21: upper absorber,
2a,21a: front portion,
2b,21b: back portion,
2c,21c: intermediate portion,
2d,21d: corner,
3,22: lower absorber,
3a,22a: front portion,
3b,22b: back portion,
3c,22c: intermediate portion,
3d,22d: corner,
3e: front end,
3f: back end,
4,23: top sheet,
4a: corner,
5,24: bottom sheet,
5a: corner,
5d,24d: wearing-article-facing surface,
6,25: rising sheet,
6a,6b: fold,
6c,25c: folded portion,
7,7a,7b,7c,26: elastic body,
8,27: hook member,
9: sheet for hook members,
X: front-back center line,
Y: left-right center line,
W: folding line

## Claims

1. An absorbent pad (1, 20) with a longitudinal direction as a front-back direction of a wearer comprising:
a liquid-retaining absorber (2, 3, 21, 22) with a longitudinal direction as the front-back direction;
a liquid-permeable top sheet (4, 23) covering an upper surface of the absorber;
a liquid-impermeable bottom sheet (5, 24) covering a lower surface of the absorber;
a wearing-article-facing surface (5d, 24d) on a side opposite to the absorber-facing surface of the bottom sheet;
left and right rising sheets (6, 25) provided with rising portions that are provided with an elastic body (7) to expand and contract in the front-back direction and rise along left and right ends of the top sheet,
the left and right rising sheets (6, 25) being fixed with an adhesive near both ends of the left-right direction and near both ends of the front-back direction of the top sheet except for the rising portions;
a hook member (8, 27) having a length in the front-back direction of 20 mm or more and 50 mm or less and a width in the left-right direction of 10 mm or more and 20 mm or less, the hook member comprising a collection of hook pieces that are detachably hooked on a wearing article; and
a hook member fixing region having a length in the front-back direction of 20 mm or more and 50 mm or less and a width in the left-right direction of more than 30 mm and less than 70 mm, and in which a plurality of the hook members are fixed parallel at intervals of less than 50 mm in the left-right direction of the absorbent pad (1, 20),
wherein the hook member fixing region is a region obtained by projecting each center region in the left-right direction of both a front end portion and a back end portion of the absorber onto the wearing-article-facing surface (5d, 24d) of the bottom sheet (5), and
wherein the absorbent pad (1, 20) is folded in half at a front-back center line (X) of the absorbent pad (1, 20) as a fold so that the hook member (8, 27) is arranged outside before use.

2. The absorbent pad according to claim 1,
wherein a sheet having the hook member (8) fixed to a surface is fixed to the hook member fixing region.

3. The absorbent pad according to claim 1,
wherein left and right side portions of the absorbent pad are folded so as to overlap the top sheet (4) at crease lines along outer edge of each tip of the left and right rising sheets that overlap the top sheet.

4. The absorbent pad according to claim 1,
wherein the hook member (8) is fixed to the hook member fixing region with an adhesive,
wherein the adhesive is applied to an entire region along a side edge farther from a left-right center line (Y) of the absorbent pad (20), and
wherein the adhesive is not applied to a region along a side edge nearer the left-right center line (Y) of the absorbent pad (20).

## Patentansprüche

1. Absorbierende Unterlage (1, 20) mit einer Längsrichtung als Vorder-Rückseite-Richtung eines Trägers, umfassend:
einem Flüssigkeit zurückhaltenden Absorber (2, 3, 21, 22) mit einer Längsrichtung als Vorder-Rückseite-Richtung;
eine flüssigkeitsdurchlässige Oberschicht (4, 23), die eine Oberseite des Absorbers bedeckt;
eine flüssigkeitsundurchlässige Unterschicht (5, 24), die eine Unterseite des Absorbers bedeckt;
eine dem Kleidungsstück zugewandte Oberfläche (5d, 24d) auf einer Seite gegenüber der dem Absorber zugewandten Oberfläche der Unterschicht;
eine linke und eine rechte aufstehende Schicht (6, 25), die mit Stehabschnitten versehen sind, die mit einem elastischen Körper (7) versehen sind, um sich in der Vorder-Rückseite-Richtung auszudehnen und zusammenzuziehen und sich entlang der linken und rechten Enden der Oberschicht zu erheben,
wobei die linke und rechte aufstehende Schicht (6, 25) mit einem Klebstoff in der Nähe beider Enden in der Links-Rechts-Richtung und in der Nähe beider Enden in der Vorder-Rückseiten-Richtung der Oberschicht mit Ausnahme der Stehabschnitte befestigt sind;
ein Hakenelement (8, 27) mit einer Länge in der Vorder-Rückseiten-Richtung von 20 mm oder mehr und 50 mm oder weniger und einer Breite in der Links-Rechts-Richtung von 10 mm oder mehr und 20 mm oder weniger, wobei das Hakenelement eine Ansammlung von Hakenstücken umfasst, die lösbar an einem Kleidungsstück eingehakt werden können; und
ein Hakenelementbefestigungsbereich mit einer Länge in der Vorder-Rückseiten-Richtung von 20 mm oder mehr und 50 mm oder weniger und einer Breite in der Links-Rechts-Richtung von mehr als 30 mm und weniger als 70 mm, und in dem mehrere Hakenelemente parallel in Abständen von weniger als 50 mm in der Links-Rechts-Richtung der absorbierenden Unterlage (1, 20) befestigt sind,
wobei der Hakenelementbefestigungsbereich ein Bereich ist, der erhalten wird, indem jeder Mittelbereich in der Links-Rechts-Richtung sowohl eines vorderen Endabschnitts als auch eines hinteren Endabschnitts des Absorbers auf die dem Kleidungsstück zugewandte Oberfläche (5d, 24d) der Unterschicht (5) projiziert wird, und
wobei die absorbierende Unterlage (1, 20) an einer Vorder-Rückseiten-Mittellinie (X) der absorbierenden Unterlage (1, 20) als Falte in der Mitte gefaltet ist, so dass das Hakenelement (8, 27) vor der Verwendung außerhalb angeordnet ist.

2. Absorbierende Unterlage gemäß Anspruch 1,
wobei eine Schicht, an deren Oberfläche das Hakenelement (8) befestigt ist, an dem Hakenelementbefestigungsbereich befestigt ist.

3. Absorbierende Unterlage gemäß Anspruch 1,
wobei die linken und rechten Seitenteile der absorbierenden Unterlage so gefaltet sind, dass sie die Oberschicht (4) an Falzlinien entlang der Außenkante jeder Spitze der linken und rechten aufstehenden Schichten, die die Oberschicht überlappen, überlappen.

4. Absorbierende Unterlage gemäß Anspruch 1,
wobei das Hakenelement (8) mit einem Klebstoff an dem Hakenelementbefestigungsbereich befestigt ist,
wobei der Klebstoff auf einen gesamten Bereich entlang einer Seitenkante aufgetragen ist, die weiter von einer Links-Rechts-Mittellinie (Y) der absorbierenden Unterlage (20) entfernt ist, und
wobei der Klebstoff nicht auf einen Bereich entlang einer Seitenkante aufgetragen wird, die näher an der Links-Rechts-Mittellinie (Y) der saugfähigen Unterlage (20) liegt.

## Revendications

1. Garniture absorbante (1, 20) avec une direction longitudinale en tant que direction avant-arrière d'une utilisatrice comprenant :
un absorbeur de retenue de liquide (2, 3, 21, 22) avec une direction longitudinale en tant que direction avant-arrière ;
une feuille supérieure perméable au liquide (4, 23) recouvrant une surface supérieure du l'absorbeur ;
une feuille inférieure imperméable au liquide (5, 24) recouvrant une surface inférieure de l'absorbeur ;
une surface orientée vers l'article d'habillement (5d, 24d) sur un côté opposé à la surface orientée vers l'absorbeur de la feuille inférieure ;
des feuilles ascendantes gauche et droite (6, 25) prévues avec des parties ascendantes qui sont prévues avec un corps élastique (7) pour se dilater et se contracter dans la direction avant-arrière et monter le long des extrémités gauche et droite de la feuille supérieure,
les feuilles ascendantes gauche et droite (6,25) étant fixées avec un adhésif à proximité des deux extrémités de la direction gauche-droite et à proximité des deux extrémités de la direction avant-arrière de la feuille supérieure, excepté pour les parties ascendantes ;
un élément de crochet (8, 27) ayant une longueur dans la direction avant-arrière de 20 mm ou plus et de 50 mm ou moins et une largeur dans la direction gauche-droite de 10 mm ou plus et de 20 mm ou moins, l'élément de crochet comprenant une pluralité de pièces de crochet qui sont accrochées de manière détachable sur un article d'habillement ; et
une région de fixation d'élément de crochet ayant une longueur dans la direction avant-arrière de 20 mm ou plus et de 50 mm ou moins et une largeur dans la direction gauche-droite supérieure à 30 mm et inférieure à 70 mm, et dans laquelle la pluralité d'éléments de crochet sont fixés en parallèle à intervalles de moins de 50 mm dans la direction gauche-droite de la garniture absorbante (1, 20),
dans laquelle la région de fixation d'élément de crochet est une région obtenue en projetant chaque région centrale dans la direction gauche-droite à la fois d'une partie d'extrémité avant et d'une partie d'extrémité arrière de l'absorbeur sur la surface orientée vers l'article d'habillement (5d, 24d) de la feuille inférieure (5), et
dans laquelle la garniture absorbante (1, 20) est pliée en deux au niveau d'une ligne centrale avant-arrière (X) de la garniture absorbante (1, 20) en tant que pli de sorte que l'élément de crochet (8, 27) est agencé à l'extérieur, avant l'utilisation.

2. Garniture absorbante selon la revendication 1,
dans laquelle une feuille ayant l'élément de crochet (8) fixé sur une surface, est fixée sur la région de fixation d'élément de crochet.

3. Garniture absorbante selon la revendication 1,
dans laquelle les parties latérales gauche et droite de la garniture absorbante sont pliées afin de recouvrir la feuille supérieure (4) au niveau de lignes de pliure le long du bord externe de chaque pointe des feuilles ascendantes gauche et droite qui recouvrent la feuille supérieure.

4. Garniture absorbante selon la revendication 1,
dans laquelle l'élément de crochet (8) est fixé sur la région de fixation d'élément de crochet avec un adhésif,
dans laquelle l'adhésif est appliqué sur toute une région le long d'un bord latéral plus éloigné d'une ligne centrale gauche-droite (Y) de la garniture absorbante (20), et
dans laquelle l'adhésif n'est pas appliqué sur une région le long d'un bord latéral plus à proximité de la ligne centrale gauche-droite (Y) de la garniture absorbante (20).
